# EUROPEAN PATENT APPLICATION

(11) **EP 1 319 709 A1**
(43) Date of publication of application: **18.06.2003**
(21) Application number: 02258488.2
(22) Date of filing: 10.12.2002
(51) Int. Cl.: C12N 9/10, C12N 5/10, A01K 67/027, C12Q 1/68, A61K 38/45

(54) **Disruption of the glutathione S-transferase-omega-1 gene**

(30) Priority: 14.12.2001 US 341483 P
(71) Applicant: Pfizer Products Inc., Groton, Connecticut 06340 (US)
(72) Inventor: Allen-Mcconkey, Melanie, Pfizer Global Res. & Dev., Groton, Connecticut 06340 (US); Audoly, Laurent Pascal, Pfizer Global Res. & Dev., Groton, Connecticut 06340 (US); Gabel, Christopher A., Pfizer Global Res. & Dev., Groton, Connecticut 06340 (US)
(74) Representative: England, Peter Michael

(57) **Abstract**

The invention features non-human mammals and animal cells that contain a targeted disruption of a glutathione S-transferase-Omega-1 gene. The invention also features methods of treating an IL-1 mediated and/or inflammation mediated disorder in a mammal comprising administering an agent that inhibits GST-Omega-1 activity.

## Description

This application claims priority under 35 U.S.C. § 120 to provisional application 60/341,483 filed 12/14/2001 which application is incorporated herein by reference in its entirety.

### Field of the Invention

The present invention features genetically-modified non-human mammals and animal cells containing a disrupted glutathione S-transferase-Omega-1 (GST-Omega-1) gene.

### Background of the Invention

The glutathione S-transferases (GSTs) are a family of phase II enzymes that utilize glutathione in biotransformation reactions that dispose of a wide variety of compounds, including chemical carcinogens, therapeutic drugs, and the products of oxidative stress (Board et al., J. Biol. Chem. 275: 24798-806, 2000).

A recently identified class of GSTs, the Omega class, has unique structural and functional characteristics as compared to the other six classes of GSTs (Board et al., *supra*; Kodym et al., J. Biol. Chem. 274: 5131-37, 1999). The human (Board et al., *supra*), mouse (Kodym et al., *supra*), and rat (Ishikawa et al., J. Biol. Chem. 273: 28708-812, 1998) GST-Omega-1 homologues have been identified.

GST-Omega-1 is widely expressed, with the highest levels of expression in the liver and kidney (Board et al., *supra*). GST-Omega-1 has been shown to act as a stress response protein involved in cellular redox homeostasis (Kodym et al., *supra*), a glutathione-dependent S-thiotransferase (Board et al., *supra*), and as a regulator of intracellular ascorbate levels (Ishikawa et al., *supra*). However, the total picture regarding the physiological effects of GST-Omega-1 action still remains to be resolved. Thus, there is a need for additional research tools, including GST-Omega-1 knockout mice, to further define the physiological role of GST-Omega-1 action, and the therapeutic implications associated with modulating GST-Omega-1 activity.

### Summary of the Invention

The present invention features genetically-modified non-human mammals and animal cells that are homozygous or heterozygous for a disrupted GST-Omega-1 gene.

In the first aspect, the invention features a genetically-modified, non-human mammal, wherein the modification results in a disrupted GST-Omega-1 gene. Preferably, the mammal is a rodent, more preferably, a mouse.

The second aspect of the invention features a genetically-modified animal cell, wherein the modification comprises a disrupted GST-Omega-1 gene. In preferred embodiments, the cell is an embryonic stem (ES) cell, an ES-like cell, and/or the cell is murine or human. In another preferred embodiment, the cell is isolated from a genetically-modified, non-human mammal containing a modification that results in a disrupted GST-Omega-1 gene.

In the third aspect, the invention features a method of identifying a gene that demonstrates modified expression as a result of modified GST-Omega-1 activity in an animal cell, said method comprising comparing the expression profile of a genetically modified animal cell, wherein the cell is homozygous for a genetic modification that disrupts the GST-Omega-1 gene, to a wild type cell.

The fourth aspect of the invention features methods of treating an IL-1 mediated and/or inflammation mediated disorder in a mammal, comprising administering an agent that inhibits GST-Omega-1 activity. Preferably, the agent is administered to treat rheumatoid arthritis, osteoarthritis, inflammatory bowel disorder, osteoporosis, or IL-1 dependent cancer.

Those skilled in the art will fully understand the terms used herein in the description and the appendant claims to describe the present invention. Nonetheless, unless otherwise provided herein, the following terms are as described immediately below.

A non-human mammal or an animal cell that is "genetically-modified" is heterozygous or homozygous for a modification that is introduced into the non-human mammal or animal cell, or into a progenitor non-human mammal or animal cell, by genetic engineering. The standard methods of genetic engineering that are available for introducing the modification include homologous recombination, viral vector gene trapping, irradiation, chemical mutagenesis, and the transgenic expression of a nucleotide sequence encoding antisense RNA alone or in combination with catalytic ribozymes. Preferred methods for genetic modification to disrupt a gene are those which modify an endogenous gene by inserting a "foreign nucleic acid sequence" into the gene locus, e.g., by homologous recombination or viral vector gene trapping. A "foreign nucleic acid sequence" is an exogenous sequence that is non-naturally occurring in the gene. This insertion of foreign DNA can occur within any region of the GST-Omega-1 gene, e.g., in an enhancer, promoter, regulator region, noncoding region, coding region, intron, or exon. The most preferred method of genetic engineering for gene disruption is homologous recombination, in which the foreign nucleic acid sequence is inserted in a targeted manner either alone or in combination with a deletion of a portion of the endogenous gene sequence.

By a GST-Omega-1 gene that is "disrupted" is meant a GST-Omega-1 gene that is genetically modified such that the cellular activity of the GST-Omega-1 polypeptide encoded by the disrupted gene is decreased or eliminated in cells that normally express a wild type version of the GST-Omega-1 gene. When the genetic modification effectively eliminates all wild type copies of the GST-Omega-1 gene in a cell (e.g., the genetically-modified, non-human mammal or animal cell is homozygous for the GST-Omega-1 gene disruption or the only wild type copy of the GST-Omega-1 gene originally present is now disrupted), the genetic modification results in a reduction in GST-Omega-1 polypeptide activity as compared to a control cell that expresses the wild type GST-Omega-1 gene. This reduction in GST-Omega-1 polypeptide activity results from either reduced GST-Omega-1 gene expression (i.e., GST-Omega-1 mRNA levels are effectively reduced resulting in reduced levels of GST-Omega-1 polypeptide) and/or because the disrupted GST-Omega-1 gene encodes a mutated polypeptide with altered, e.g., reduced, function as compared to a wild type GST-Omega-1 polypeptide. Preferably, the activity of GST-Omega-1 polypeptide in the genetically-modified, non-human mammal or animal cell is reduced to 50% or less of wild type levels, more preferably, to 25% or less, and, even more preferably, to 10% or less of wild type levels. Most preferably, the GST-Omega-1 gene disruption results in non-detectable GST-Omega-1 activity.

By a "genetically-modified, non-human mammal" containing a disrupted GST-Omega-1 gene is meant a non-human mammal that is originally produced, for example, by creating a blastocyst or embryo carrying the desired genetic modification and then implanting the blastocyst or embryo in a foster mother for *in utero* development. The genetically-modified blastocyst or embryo can be made, in the case of mice, by implanting a genetically-modified embryonic stem (ES) cell into a mouse blastocyst or by aggregating ES cells with tetraploid embryos. Alternatively, various species of genetically-modified embryos can be obtained by nuclear transfer. In the case of nuclear transfer, the donor cell is a somatic cell or a pluripotent stem cell, and it is engineered to contain the desired genetic modification that disrupts the GST-Omega-1 gene. The nucleus of this cell is then transferred into a fertilized or parthenogenetic oocyte that is enucleated; the resultant embryo is reconstituted and developed into a blastocyst. A genetically-modified blastocyst produced by either of the above methods is then implanted into a foster mother according to standard methods well known to those skilled in the art. A "genetically-modified, non-human mammal" includes all progeny of the non-human mammals created by the methods described above, provided that the progeny inherit at least one copy of the genetic modification that disrupts the GST-Omega-1 gene. It is preferred that all somatic cells and germline cells of the genetically-modified non-human mammal contain the modification. Preferred non-human mammals that are genetically-modified to contain a disrupted GST-Omega-1 gene include rodents, such as mice and rats, cats, dogs, rabbits, guinea pigs, hamsters, sheep, pigs, and ferrets.

By a "genetically-modified animal cell" containing a disrupted GST-Omega-1 gene is meant an animal cell, including a human cell, created by genetic engineering to contain a disrupted GST-Omega-1 gene, as well as daughter cells that inherit the disrupted GST-Omega-1 gene. These cells may be genetically-modified in culture according to any standard method known in the art. As an alternative to genetically modifying the cells in culture, non-human mammalian cells may also be isolated from a genetically-modified, non-human mammal that contains a GST-Omega-1 gene disruption. The animal cells of the invention may be obtained from primary cell or tissue preparations as well as culture-adapted, tumorigenic, or transformed cell lines. These cells and cell lines are derived, for example, from endothelial cells, epithelial cells, islets, neurons and other neural tissue-derived cells, mesothelial cells, osteocytes, lymphocytes, chondrocytes, hematopoietic cells, immune cells, cells of the major glands or organs (e.g., testicle, liver, lung, heart, stomach, pancreas, kidney, and skin), muscle cells (including cells from skeletal muscle, smooth muscle, and cardiac muscle), exocrine or endocrine cells, fibroblasts, and embryonic and other totipotent or pluripotent stem cells (e.g., ES cells, ES-like cells, and embryonic germline (EG) cells, and other stem cells, such as progenitor cells and tissue-derived stem cells). The preferred genetically-modified cells are ES cells, more preferably, mouse or rat ES cells, and, most preferably, human ES cells.

By an "ES cell" or an "ES-like cell" is meant a pluripotent stem cell derived from an embryo, from a primordial germ cell, or from a teratocarcinoma, that is capable of indefinite self renewal as well as differentiation into cell types that are representative of all three embryonic germ layers.

By "modified GST-Omega-1 activity" is meant a change in the activity of the GST-Omega-1 enzyme as a result of genetic manipulation of the GST-Omega-1 gene that causes a change in the level of GST-Omega-1 polypeptide in a cell, or as the result of administration of a pharmacological agent that agonizes or antagonizes GST-Omega-1 activity.

By "modified expression" of a gene is meant the up-regulation or down-regulation of the gene as compared to the control.

By "inhibits GST-Omega-1 activity" is meant decreases GST-Omega-1 activity produced in a cell.

Other features and advantages of the invention will be apparent from the following detailed description and from the claims. While the invention is described in connection with specific embodiments, it will be understood that other changes and modifications that may be practiced are also part of this invention and are also within the scope of the appendant claims. This application is intended to cover any equivalents, variations, uses, or adaptations of the invention that follow, in general, the principles of the invention, including departures from the present disclosure that come within known or customary practice within the art, and that are able to be ascertained without undue experimentation. Additional guidance with respect to making and using nucleic acids and polypeptides is found in standard textbooks of molecular biology, protein science, and immunology (see, e.g., Davis et al., *Basic Methods in Molecular Biology*, Elsevir Sciences Publishing, Inc., New York, NY,1986; Hames et al., *Nucleic Acid Hybridization*, IL Press, 1985; Molecular Cloning, Sambrook et al., *Current Protocols in Molecular Biology*, Eds. Ausubel et al., John Wiley and Sons; *Current Protocols in Human Genetics*, Eds. Dracopoli et al., John Wiley and Sons; *Current Protocols in Protein Science*, Eds. John E. Coligan et al., John Wiley and Sons; and *Current Protocols in Immunology*, Eds. John E. Coligan et al., John Wiley and Sons). All publications mentioned herein are incorporated by reference in their entireties.

### Brief Description of the Figures

Fig. 1 is a schematic depicting the GST-Omega-1 gene targeting vector, the location for homologous recombination of the vector in the endogenous murine GST-Omega-1 gene, and the polymerase chain reaction (PCR) strategy used to verify gene targeting.

Fig. 2 shows the results of polymerase chain reaction (PCR)-based genotyping of wild-type (+/+), heterozygote (+/-), and knockout (-/-) mice with respect to the disrupted GST-Omega-1 allele. Southern analysis following Spel digestion revealed a 12 kb Spel fragment from the wild type allele and a 9.5 kb fragment from the GST-Omega-1 allele that underwent targeted disruption.

Fig. 3 shows the reverse transcription-PCR analysis performed on RNA isolated from various tissues

Fig. 4 is a graph showing the severity of collagen-induced arthritis in wild type (WT) and GST-Omega-1 knockout (KO) mice over time.

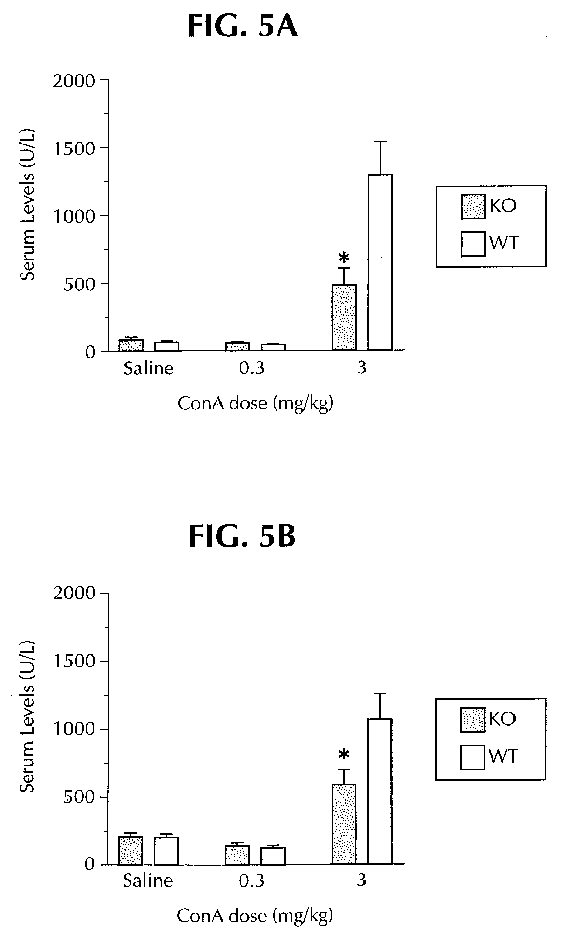

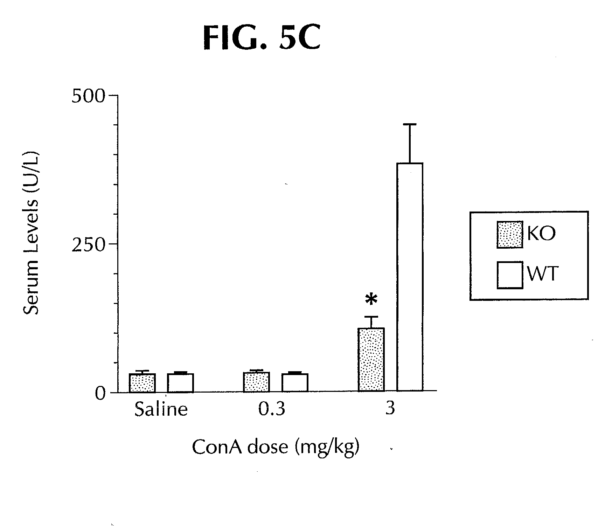

Fig. 5 is a graph showing the effects of concanavalin A injection on liver enzymes in WT and GST-Omega-1 KO mice eight hours after injection. Fig. 5A shows effects on alanine transaminase; Fig. 5B shows effects on aspartate transaminase; and Fig. 5C shows effects on sorbitol dehydrogenase.

### Detailed Description of the Invention

### Genetically-Modified Non-human Mammals and Animal Cells Containing a Disrupted GST-Omega-1 Gene

### 1. Genetically-Modified Non-human Mammals and Animal Cells

The genetically-modified, non-human mammals and genetically-modified animal cells, including human cells, of the invention are heterozygous or homozygous for a modification that disrupts the GST-Omega-1 gene. The animal cells may be derived by genetically engineering cells in culture, or, in the case of non-human mammalian cells, the cells may be isolated from genetically-modified, non-human mammals.

The GST-Omega-1 gene locus is disrupted by one of the several techniques for genetic modification known in the art, including chemical mutagenesis (Rinchik, Trends in Genetics 7: 15-21, 1991, Russell, Environmental & Molecular Mutagenesis 23 (Suppl. 24): 23-29, 1994), irradiation (Russell, *supra*), transgenic expression of GST-Omega-1 gene antisense RNA, either alone or in combination with a catalytic RNA ribozyme sequence (Luyckx et al., Proc. Natl. Acad. Sci. 96: 12174-79, 1999; Sokol et al., Transgenic Research 5: 363-71, 1996; Efrat et al., Proc. Natl. Acad. Sci. USA 91: 2051-55, 1994; Larsson et al., Nucleic Acids Research 22: 2242-48, 1994) and, as further discussed below, the disruption of the GST-Omega-1 gene by the insertion of a foreign nucleic acid sequence into the GST-Omega-1 gene locus. Preferably, the foreign sequence is inserted by homologous recombination or by the insertion of a viral vector. Most preferably, the method of GST-Omega-1 gene disruption to create the genetically modified non-human mammals and animal cells of the invention is homologous recombination and includes a deletion of a portion of the endogenous GST-Omega-1 gene sequence.

The integration of the foreign sequence disrupts the GST-Omega-1 gene through one or more of the following mechanisms: by interfering with the GST-Omega-1 gene transcription or translation process (e.g., by interfering with promoter recognition, or by introducing a transcription termination site or a translational stop codon into the GST-Omega-1 gene); or by distorting the GST-Omega-1 gene coding sequence such that it no longer encodes a GST-Omega-1 polypeptide with normal function (e.g., by inserting a foreign coding sequence into the GST-Omega-1 gene coding sequence, by introducing a frameshift mutation or amino acid(s) substitution, or, in the case of a double crossover event, by deleting a portion of the GST-Omega-1 gene coding sequence that is required for expression of a functional GST-Omega-1 protein).

To insert a foreign sequence into a GST-Omega-1 gene locus in the genome of a cell to create the genetically modified non-human mammals and animal cells of the invention based upon the present description, the foreign DNA sequence is introduced into the cell according to a standard method known in the art such as electroporation, calcium-phosphate precipitation, retroviral infection, microinjection, biolistics, liposome transfection, DEAE-dextran transfection, or transferrinfection (see, e.g., Neumann et al., EMBO J. 1: 841-845, 1982; Potter et al., Proc. Natl. Acad. Sci USA 81: 7161-65, 1984; Chu et al., Nucleic Acids Res. 15: 1311-26, 1987; Thomas and Capecchi, Cell 51: 503-12, 1987; Baum et al., Biotechniques 17: 1058-62, 1994; Biewenga et al., J. Neuroscience Methods 71: 67-75, 1997; Zhang et al., Biotechniques 15: 868-72, 1993; Ray and Gage, Biotechniques 13: 598-603, 1992; Lo, Mol. Cell. Biol. 3: 1803-14, 1983; Nickoloff et al., Mol. Biotech. 10: 93-101, 1998; Linney et al., Dev. Biol. (Orlando) 213: 207-16, 1999; Zimmer and Gruss, Nature 338: 150-153, 1989; and Robertson et al., Nature 323: 445-48, 1986). The preferred method for introducing foreign DNA into a cell is electroporation.

### 2. Homologous Recombination

The method of homologous recombination targets the GST-Omega-1 gene for disruption by introducing a GST-Omega-1 gene targeting vector into a cell containing a GST-Omega-1 gene. The ability of the vector to target the GST-Omega-1 gene for disruption stems from using a nucleotide sequence in the vector that is homologous, i.e., related, to the GST-Omega-1 gene. This homology region facilitates hybridization between the vector and the endogenous sequence of the GST-Omega-1 gene. Upon hybridization, the probability of a crossover event between the targeting vector and genomic sequences greatly increases. This crossover event results in the integration of the vector sequence into the GST-Omega-1 gene locus and the functional disruption of the GST-Omega-1 gene.

General principles regarding the construction of vectors used for targeting are reviewed in Bradley et al. (Biotechnol. 10: 534, 1992). Two different types of vector can be used to insert DNA by homologous recombination: an insertion vector or a replacement vector. An insertion vector is circular DNA which contains a region of GST-Omega-1 gene homology with a double stranded break. Following hybridization between the homology region and the endogenous GST-Omega-1 gene, a single crossover event at the double stranded break results in the insertion of the entire vector sequence into the endogenous gene at the site of crossover.

The more preferred vector to create the genetically modified non-human mammals and animals cells of the invention by homologous recombination is a replacement vector, which is colinear rather than circular. Replacement vector integration into the GST-Omega-1 gene requires a double crossover event, i.e. crossing over at two sites of hybridization between the targeting vector and the GST-Omega-1 gene. This double crossover event results in the integration of a vector sequence that is sandwiched between the two sites of crossover into the GST-Omega-1 gene and the deletion of the corresponding endogenous GST-Omega-1 gene sequence that originally spanned between the two sites of crossover (see, e.g., Thomas and Capecchi et al., Cell 51: 503-12, 1987; Mansour et al., Nature 336: 348-52, 1988; Mansour et al., Proc. Natl. Acad. Sci. USA 87: 7688-7692, 1990; and Mansour, GATA 7: 219-227, 1990).

A region of homology in a targeting vector used to create the genetically modified non-human mammals and animal cells of the invention is generally at least 100 nucleotides in length. Most preferably, the homology region is at least 1-5 kilobases (kb) in length. Although there is no demonstrated minimum length or minimum degree of relatedness required for a homology region, targeting efficiency for homologous recombination generally corresponds with the length and the degree of relatedness between the targeting vector and the GST-Omega-1 gene locus. In the case where a replacement vector is used, and a portion of the endogenous GST-Omega-1 gene is deleted upon homologous recombination, an additional consideration is the size of the deleted portion of the endogenous GST-Omega-1 gene. If this portion of the endogenous GST-Omega-1 gene is greater than 1 kb in length, then a targeting cassette with regions of homology that are longer than 1 kb is recommended to enhance the efficiency of recombination. Further guidance regarding the selection and use of sequences effective for homologous recombination, based on the present description, is described in the literature (see, e.g., Deng and Capecchi, Mol. Cell. Biol. 12: 3365-3371, 1992; Bollag et al., Annu. Rev. Genet. 23: 199-225, 1989; and Waldman and Liskay, Mol. Cell. Biol. 8: 5350-5357, 1988).

As those skilled in the art will recognize based upon the present invention, a wide variety of cloning vectors may be used as vector backbones in the construction of the GST-Omega-1 gene targeting vectors of the present invention, including pBluescript-related plasmids (e.g., Bluescript KS+11), pQE70, pQE60, pQE-9, pBS, pD10, phagescript, phiX174, pBK Phagemid, pNH8A, pNH16a, pNH18Z, pNH46A, ptrc99a, pKK223-3, pKK233-3, pDR540, and pRIT5 PWLNEO, pSV2CAT, pXT1, pSG (Stratagene), pSVK3, PBPV, PMSG, and pSVL, pBR322 and pBR322-based vectors, pMB9, pBR325, pKH47, pBR328, pHC79, phage Charon 28, pKB11, pKSV-10, pK19 related plasmids, pUC plasmids, and the pGEM series of plasmids. These vectors are available from a variety of commercial sources (e.g., Boehringer Mannheim Biochemicals, Indianapolis, IN; Qiagen, Valencia, CA; Stratagene, La Jolla, CA; Promega, Madison, WI; and New England Biolabs, Beverly, MA). However, any other vectors, e.g. plasmids, viruses, or parts thereof, may be used as long as they are replicable and viable in the desired host. The vector may also comprise sequences which enable it to replicate in the host whose genome is to be modified. The use of such a vector can expand the interaction period during which recombination can occur, increasing the efficiency of targeting (see *Molecular Biology*, ed. Ausubel et al, Unit 9.16, Fig. 9.16.1).

The specific host employed for propagating the targeting vectors of the present invention is not critical. Examples include *E. coli* K12 RR1 (Bolivar et al., Gene 2: 95, 1977), *E. coli* K12 HB101 (ATCC No. 33694), *E. coli* MM21 (ATCC No. 336780), *E. coli* DH1 (ATCC No. 33849), *E. coli* strain DH5α, and *E. coli* STBL2. Alternatively, hosts such as *C. cerevisiae* or *B. subtilis* can be used. The above-mentioned hosts are available commercially (e.g., Stratagene, La Jolla, CA; and Life Technologies, Rockville, MD).

To create the targeting vector, a GST-Omega-1 gene targeting construct is added to an above-described vector backbone. The GST-Omega-1 gene targeting constructs of the invention have at least one GST-Omega-1 gene homology region. To make the GST-Omega-1 gene homology regions, a GST-Omega-1 genomic or cDNA sequence is used as a basis for producing PCR primers. These primers are used to amplify the desired region of the GST-Omega-1 sequence by high fidelity PCR amplification (Mattila et al., Nucleic Acids Res. 19: 4967, 1991; Eckert and Kunkei 1: 17, 1991; and U.S. Pat. No. 4,683, 202). The genomic sequence is obtained from a genomic clone library or from a preparation of genomic DNA, preferably from the animal species that is to be targeted for GST-Omega-1 gene disruption. The GST-Omega-1 cDNA sequence can be used in making a GST-Omega-1 targeting vector (murine, Genbank U80819; human, Genbank AF212303; rat, Genbank AB008807).

Preferably, the targeting constructs of the invention also include an exogenous nucleotide sequence encoding a positive marker protein. The stable expression of a positive marker after vector integration confers an identifiable characteristic on the cell, ideally, without compromising cell viability. Therefore, in the case of a replacement vector, the marker gene is positioned between two flanking homology regions so that it integrates into the GST-Omega-1 gene following the double crossover event in a manner such that the marker gene is positioned for expression after integration.

It is preferred that the positive marker protein is a selectable protein; the stable expression of such a protein in a cell confers a selectable phenotypic characteristic, i.e., the characteristic enhances the survival of the cell under otherwise lethal conditions. Thus, by imposing the selectable condition, one can isolate cells that stably express the positive selectable marker-encoding vector sequence from other cells that have not successfully integrated the vector sequence on the basis of viability. Examples of positive selectable marker proteins (and their agents of selection) include neo (G418 or kanomycin), hyg (hygromycin), hisD (histidinol), gpt (xanthine), ble (bleomycin), and hprt (hypoxanthine) (see, e.g., Capecchi and Thomas, U.S. Pat. No. 5,464,764, and Capecchi, Science 244: 1288-92, 1989). Other positive markers that may also be used as an alternative to a selectable marker include reporter proteins such as β-galactosidase, firefly luciferase, or GFP (see, e.g., *Current Protocols in Cytometry*, Unit 9.5, and *Current Protocols in Molecular Biology*, Unit 9.6, John Wiley & Sons, New York, NY, 2000).

The above-described positive selection step does not distinguish between cells that have integrated the vector by targeted homologous recombination at the GST-Omega-1 gene locus versus random, non-homologous integration of vector sequence into any chromosomal position. Therefore, when using a replacement vector for homologous recombination to make the genetically modified non-human mammals and animal cells of the invention, it is also preferred to include a nucleotide sequence encoding a negative selectable marker protein. Expression of a negative selectable marker causes a cell expressing the marker to lose viability when exposed to a certain agent (i.e., the marker protein becomes lethal to the cell under certain selectable conditions). Examples of negative selectable markers (and their agents of lethality) include herpes simplex virus thymidine kinase (gancyclovir or 1,2-deoxy-2-fluoro-*a*-d-arabinofuransyl-5-iodouracil), Hprt (6-thioguanine or 6-thioxanthine), and diphtheria toxin, ricin toxin, and cytosine deaminase (5-fluorocytosine).

The nucleotide sequence encoding the negative selectable marker is positioned outside of the two homology regions of the replacement vector. Given this positioning, cells will only integrate and stably express the negative selectable marker if integration occurs by random, non-homologous recombination; homologous recombination between the GST-Omega-1 gene and the two regions of homology in the targeting construct excludes the sequence encoding the negative selectable marker from integration. Thus, by imposing the negative condition, cells that have integrated the targeting vector by random, non-homologous recombination lose viability.

The above-described combination of positive and negative selectable markers is preferred in a targeting construct used to make the genetically modified non-human mammals and animal cells of the invention because a series of positive and negative selection steps can be designed to more efficiently select only those cells that have undergone vector integration by homologous recombination, and, therefore, have a potentially disrupted GST-Omega-1 gene. Further examples of positive-negative selection schemes, selectable markers, and targeting constructs are described, for example, in U.S. Pat. No. 5,464,764, WO 94/06908, U.S. Pat. No. 5,859,312, and Valancius and Smithies, Mol. Cell. Biol. 11: 1402, 1991.

In order for a marker protein to be stably expressed upon vector integration, the targeting vector may be designed so that the marker coding sequence is operably linked to the endogenous GST-Omega-1 gene promoter upon vector integration. Expression of the marker is then driven by the GST-Omega-1 gene promoter in cells that normally express the GST-Omega-1 gene. Alternatively, each marker in the targeting construct of the vector may contain its own promoter that drives expression independent of the GST-Omega-1 gene promoter. This latter scheme has the advantage of allowing for expression of markers in cells that do not typically express the GST-Omega-1 gene (Smith and Berg, Cold Spring Harbor Symp. Quant. Biol. 49: 171, 1984; Sedivy and Sharp, Proc. Natl. Acad. Sci. (USA) 86: 227, 1989; Thomas and Capecchi, Cell 51: 503, 1987).

Exogenous promoters that can be used to drive marker gene expression include cell-specific or stage-specific promoters, constitutive promoters, and inducible or regulatable promoters. Non-limiting examples of these promoters include the herpes simplex thymidine kinase promoter, cytomegalovirus (CMV) promoter/enhancer, SV40 promoters, PGK promoter, PMC1-neo, metallothionein promoter, adenovirus late promoter, vaccinia virus 7.5K promoter, avian beta globin promoter, histone promoters (e.g., mouse histone H3-614), beta actin promoter, neuron-specific enolase, muscle actin promoter, and the cauliflower mosaic virus 35S promoter (see generally, Sambrook et al., *Molecular Cloning*, Vols. I-III, Cold Spring Harbor Laboratory Press, Cold Spring Harbor, NY, 1989, and *Current Protocols in Molecular Biology*, John Wiley & Sons, New York, NY, 2000; Stratagene, La Jolla, CA).

To confirm whether cells have integrated the vector sequence into the targeted GST-Omega-1 gene locus while making the genetically modified non-human mammals and animal cells of the invention, primers or genomic probes that are specific for the desired vector integration event can be used in combination with PCR or Southern blot analysis to identify the presence of the desired vector integration into the GST-Omega-1 gene locus (Erlich et al., Science 252: 1643-51, 1991; Zimmer and Gruss, Nature 338: 150, 1989; Mouellic et al., Proc. Natl. Acad. Sci. (USA) 87: 4712, 1990; and Shesely et al., Proc. Natl. Acad. Sci. (USA) 88: 4294, 1991).

### 3. Gene Trapping

Another method available for inserting a foreign nucleic acid sequence into the GST-Omega-1 gene locus to disrupt the GST-Omega-1 gene, based on the present description, is gene trapping. This method takes advantage of the cellular machinery present in all mammalian cells that splices exons into mRNA to insert a gene trap vector coding sequence into a gene in a random fashion. Once inserted, the gene trap vector creates a mutation that may disrupt the trapped GST-Omega-1 gene. In contrast to homologous recombination, this system for mutagenesis creates largely random mutations. Thus, to obtain a genetically-modified cell that contains a disrupted GST-Omega-1 gene, cells containing this particular mutation must be identified and selected from a pool of cells that contain random mutations in a variety of genes.

Gene trapping systems and vectors have been described for use in genetically modifying murine cells and other cell types (see, e.g., Allen et al., Nature 333: 852-55, 1988; Bellen et al., Genes Dev. 3: 1288-1300, 1989; Bier et al., Genes Dev. 3: 1273-1287, 1989; Bonnerot et al., J. Virol. 66: 4982-91, 1992; Brenner et al., Proc. Nat. Acad. Sci. USA 86: 5517-21, 1989; Chang et al., Virology 193: 737-47, 1993; Friedrich and Soriano, Methods Enzymol. 225: 681-701, 1993; Friedrich and Soriano, Genes Dev. 5: 1513-23, 1991; Goff, Methods Enzymol. 152: 469-81, 1987; Gossler et al., Science 244: 463-65, 1989; Hope, Develop. 113: 399-408, 1991; Kerr et al., Cold Spring Harb. Symp. Quant. Biol. 2: 767-776, 1989; Reddy et al., J. Virol. 65: 1507-1515, 1991; Reddy et al., Proc. Natl. Acad. Sci. U.S.A. 89: 6721-25, 1992; Skarnes et al., Genes Dev. 6: 903-918, 1992; von Melchner and Ruley, J. Virol. 63: 3227-3233, 1989; and Yoshida et al., Transgen. Res. 4: 277-87, 1995).

Promoter trap, or 5', vectors contain, in 5' to 3' order, a splice acceptor sequence followed by an exon, which is typically characterized by a translation initiation codon and open reading frame and/or an internal ribosome entry site. In general, these promoter trap vectors do not contain promoters or operably linked splice donor sequences. Consequently, after integration into the cellular genome of the host cell, the promoter trap vector sequence intercepts the normal splicing of the upstream gene and acts as a terminal exon. Expression of the vector coding sequence is dependent upon the vector integrating into an intron of the disrupted gene in the proper reading frame. In such a case, the cellular splicing machinery splices exons from the trapped gene upstream of the vector coding sequence (Zambrowicz et al., WO 99/50426 and U.S. Pat. No. 6,080,576).

An alternative method for producing an effect similar to the above-described promoter trap vector is a vector that incorporates a nested set of stop codons present in, or otherwise engineered into, the region between the splice acceptor of the promoter trap vector and the translation initiation codon or polyadenylation sequence. The coding sequence can also be engineered to contain an independent ribosome entry site (IRES) so that the coding sequence will be expressed in a manner largely independent of the site of integration within the host cell genome. Typically, but not necessarily, an IRES is used in conjunction with a nested set of stop codons.

Another type of gene trapping scheme uses a 3' gene trap vector. This type of vector contains, in operative combination, a promoter region, which mediates expression of an adjoining coding sequence, the coding sequence, and a splice donor sequence that defines the 3' end of the coding sequence exon. After integration into a host cell genome, the transcript expressed by the vector promoter is spliced to a splice acceptor sequence from the trapped gene that is located downstream of the integrated gene trap vector sequence. Thus, the integration of the vector results in the expression of a fusion transcript comprising the coding sequence of the 3' gene trap cassette and any downstream cellular exons, including the terminal exon and its polyadenylation signal. When such vectors integrate into a gene, the cellular splicing machinery splices the vector coding sequence upstream of the 3' exons of the trapped gene. One advantage of such vectors is that the expression of the 3' gene trap vectors is driven by a promoter within the gene trap cassette and does not require integration into a gene that is normally expressed in the host cell (Zambrowicz et al., WO 99/50426 and U.S. Pat. No. 6,080,576). Examples of transcriptional promoters and enhancers that may be incorporated into the 3' gene trap vector include those discussed above with respect to targeting vectors.

The viral vector backbone used as the structural component for the promoter or 3' gene trap vector may be selected from a wide range of vectors that can be inserted into the genome of a target cell. Suitable backbone vectors include, but are not limited to, herpes simplex virus vectors, adenovirus vectors, adeno-associated virus vectors, retroviral vectors, lentiviral vectors, pseudorabies virus, alpha-herpes virus vectors, and the like. A thorough review of viral vectors, in particular, viral vectors suitable for modifying nonreplicating cells and how to use such vectors in conjunction with the expression of an exogenous polynucleotide sequence, can be found in *Viral Vectors: Gene Therapy and Neuroscience Applications*, Eds. Caplitt and Loewy, Academic Press, San Diego, 1995.

Preferably, retroviral vectors are used for gene trapping. These vectors can be used in conjunction with retroviral packaging cell lines such as those described in U.S. Patent No. 5,449,614. Where non-murine mammalian cells are used as target cells for genetic modification, amphotropic or pantropic packaging cell lines can be used to package suitable vectors (Ory et al., Proc. Natl. Acad. Sci., USA 93: 11400-11406, 1996). Representative retroviral vectors that can be adapted to create the presently described 3' gene trap vectors are described, for example, in U.S. Pat. No. 5,521,076.

The gene trapping vectors may contain one or more of the positive marker genes discussed above with respect to targeting vectors used for homologous recombination. Similar to their use in targeting vectors, these positive markers are used in gene trapping vectors to identify and select cells that have integrated the vector into the cell genome. The marker gene may be engineered to contain an independent ribosome entry site (IRES) so that the marker will be expressed in a manner largely independent of the location in which the vector has integrated into the target cell genome.

Given that gene trap vectors will integrate into the genome of infected host cells in a fairly random manner, a genetically-modified cell having a disrupted GST-Omega-1 gene must be identified from a population of cells that have undergone random vector integration. Preferably, the genetic modifications in the population of cells are of sufficient randomness and frequency such that the population represents mutations in essentially every gene found in the cell's genome, making it likely that a cell with a disrupted GST-Omega-1 gene will be identified from the population (see Zambrowicz et al., WO 99/50426; Sands et al., WO 98/14614 and U.S. Pat. No. 6,080,576).

Individual mutant cell lines containing a disrupted GST-Omega-1 gene are identified in a population of mutated cells using, for example, reverse transcription and polymerase chain reaction (PCR) to identify a mutation in a GST-Omega-1 gene sequence. This process can be streamlined by pooling clones. For example, to find an individual clone containing a disrupted GST-Omega-1 gene, RT-PCR is performed using one primer anchored in the gene trap vector and the other primer located in the GST-Omega-1 gene sequence. A positive RT-PCR result indicates that the vector sequence is encoded in the GST-Omega-1 gene transcript, indicating that GST-Omega-1 gene has been disrupted by a gene trap integration event (see, e.g., Sands et al., WO 98/14614, U.S. Pat. No. 6,080,576).

### 4. Temporal, Spatial, and Inducible GST-Omega-1 Gene Disruptions

In certain embodiments of the present invention, a functional disruption of the endogenous GST-Omega-1 gene occurs at specific developmental or cell cycle stages (temporal disruption) or in specific cell types (spatial disruption). in other embodiments, the GST-Omega-1 gene disruption is inducible when certain conditions are present. A recombinase excision system, such as a Cre-Lox system, may be used to activate or inactivate the GST-Omega-1 gene at a specific developmental stage, in a particular tissue or cell type, or under particular environmental conditions. Generally, methods utilizing Cre-Lox technology are carried out as described by Torres and Kuhn, *Laboratory Protocols for Conditional Gene Targeting*, Oxford University Press, 1997. Methodology similar to that described for the Cre-Lox system can also be employed utilizing the FLP-FRT system. Further guidance regarding the use of recombinase excision systems for conditionally disrupting genes by homologous recombination or viral insertion is provided, for example, in U.S. Pat. No. 5,626,159, U.S. Pat. No. 5,527,695, U.S. Pat. No. 5,434,066, WO 98/29533, U.S. Pat. No. 6,228,639, Orban et al., Proc. Nat. Acad. Sci. USA 89: 6861-65, 1992; O'Gorman et al., Science 251: 1351-55, 1991; Sauer et al., Nucleic Acids Research 17: 147-61, 1989; Barinaga, Science 265: 26-28, 1994; and Akagi et al., Nucleic Acids Res. 25: 1766-73, 1997. More than one recombinase system can be used to genetically modify a non-human mammal or animal cell of the present invention.

When using homologous recombination to disrupt the GST-Omega-1 gene in a temporal, spatial, or inducible fashion, using a recombinase system such as the Cre-Lox system, a portion of the GST-Omega-1 gene coding region is replaced by a targeting construct comprising the GST-Omega-1 gene coding region flanked by loxP sites. Non-human mammals and animal cells carrying this genetic modification contain a functional, loxP-flanked GST-Omega-1 gene. The temporal, spatial, or inducible aspect of the GST-Omega-1 gene disruption is caused by the expression pattern of an additional transgene, a Cre recombinase transgene, that is expressed in the non-human mammal or animal cell under the control of the desired spatially-regulated, temporally-regulated, or inducible promoter, respectively. A Cre recombinase targets the loxP sites for recombination. Therefore, when Cre expression is activated, the LoxP sites undergo recombination to excise the sandwiched GST-Omega-1 gene coding sequence, resulting in a functional disruption of the GST-Omega-1 gene (Rajewski et al., J. Clin. Invest. 98: 600-03, 1996; St.-Onge et al., Nucleic Acids Res. 24: 3875-77, 1996; Agah et al., J. Clin. Invest. 100: 169-79, 1997; Brocard et al., Proc. Natl. Acad. Sci. USA 94: 14559-63, 1997; Feil et al., Proc. Natl. Acad. Sci. USA 93: 10887-90, 1996; and Kühn et al., Science 269: 1427-29, 1995).

A cell containing both a Cre recombinase transgene and loxP-flanked GST-Omega-1 gene can be generated through standard transgenic techniques or, in the case of genetically-modified, non-human mammals, by crossing genetically-modified, non-human mammals wherein one parent contains a loxP flanked GST-Omega-1 gene and the other contains a Cre recombinase transgene under the control of the desired promoter. Further guidance regarding the use of recombinase systems and specific promoters to temporally, spatially, or conditionally disrupt the GST-Omega-1 gene is found, for example, in Sauer, Meth. Enz. 225: 890-900, 1993, Gu et al., Science 265: 103-06, 1994, Araki et al., J. Biochem. 122: 977-82, 1997, Dymecki, Proc. Natl. Acad. Sci. 93: 6191-96, 1996, and Meyers et al., Nature Genetics 18: 136-41, 1998.

An inducible disruption of the GST-Omega-1 gene can also be achieved by using a tetracycline responsive binary system (Gossen and Bujard, Proc. Natl. Acad. Sci. USA 89: 5547-51, 1992). This system involves genetically modifying a cell to introduce a Tet promoter into the endogenous GST-Omega-1 gene regulatory element and a transgene expressing a tetracycline-controllable repressor (TetR). In such a cell, the administration of tetracycline activates the TetR which, in turn, inhibits GST-Omega-1 gene expression and, therefore, disrupts the GST-Omega-1 gene (St.-Onge et al., Nucleic Acids Res. 24: 3875-77, 1996, U.S. Patent No. 5,922,927).

The above-described systems for temporal, spatial, and inducible disruptions of the GST-Omega-1 gene can also be adopted when using gene trapping as the method of genetic modification, for example, as described, in WO 98/29533 and U.S. Pat. No. 6,288,639, for creating the genetically modified non-human mammals and animal cells of the invention.

### 5. Creating Genetically-Modified, Non-human Mammals and Animal Cells

The above-described methods for genetic modification can be used to disrupt a GST-Omega-1 gene in virtually any type of somatic or stem cell derived from an animal to create the genetically modified animal cells of the invention. Genetically-modified animal cells of the invention include, but are not limited to, mammalian cells, including human cells, and avian cells. These cells may be derived from genetically engineering any animal cell line, such as culture-adapted, tumorigenic, or transformed cell lines, or they may be isolated from a genetically-modified, non-human mammal carrying the desired GST-Omega-1 genetic modification.

The cells may be heterozygous or homozygous for the disrupted GST-Omega-1 gene. To obtain cells that are homozygous for the GST-Omega-1 gene disruption (GST-Omega-1-/-), direct, sequential targeting of both alleles can be performed. This process can be facilitated by recycling a positive selectable marker. According to this scheme the nucleotide sequence encoding the positive selectable marker is removed following the disruption of one allele using the Cre-Lox P system. Thus, the same vector can be used in a subsequent round of targeting to disrupt the second GST-Omega-1 gene allele (Abuin and Bradley, Mol. Cell. Biol. 16: 1851-56, 1996; Sedivy et al., T.I.G. 15: 88-90, 1999; Cruz et al., Proc. Natl. Acad. Sci. (USA) 88: 7170-74, 1991; Mortensen et al., Proc. Natl. Acad. Sci. (USA) 88: 7036-40, 1991; te Riele et al., Nature (London) 348: 649-651, 1990).

An alternative strategy for obtaining ES cells that are GST-Omega-1-/- is the homogenotization of cells from a population of cells that is heterozygous for the GST-Omega-1 gene disruption (GST-Omega-1+/-). The method uses a scheme in which GST-Omega-1+/- targeted clones that express a selectable drug resistance marker are selected against a very high drug concentration; this selection favors cells that express two copies of the sequence encoding the drug resistance marker and are, therefore, homozygous for the GST-Omega-1 gene disruption (Mortensen et al., Mol. Cell. Biol. 12: 2391-95, 1992). In addition, genetically-modified animal cells can be obtained from genetically-modified GST-Omega-1-/- non-human mammals that are created by mating non-human mammals that are GST-Omega-1+/- in germline cells, as further discussed below.

Following the genetic modification of the desired cell or cell line, the GST-Omega-1 gene locus can be confirmed as the site of modification by PCR analysis according to standard PCR or Southern blotting methods known in the art (see, e.g., U.S. Pat. No. 4,683,202; and Erlich et al., Science 252: 1643, 1991). Further verification of the functional disruption of the GST-Omega-1 gene may also be made if GST-Omega-1 gene messenger RNA (mRNA) levels and/or GST-Omega-1 polypeptide levels are reduced in cells that normally express the GST-Omega-1 gene. Measures of GST-Omega-1 gene mRNA levels may be obtained by using reverse transcriptase mediated polymerase chain reaction (RT-PCR), Northern blot analysis, or *in situ* hybridization. The quantification of GST-Omega-1 polypeptide levels produced by the cells can be made, for example, by standard immunoassay methods known in the art. Such immunoassays include, but are not limited to, competitive and non-competitive assay systems using techniques such as RIAs (radioimmunoassays), ELISAs (enzyme-linked immunosorbent assays), "sandwich" immunoassays, immunoradiometric assays, gel diffusion precipitin reactions, immunodiffusion assays, *in situ* immunoassays (using, for example, colloidal gold, enzymatic, or radioisotope labels), Western blots, 2-dimensional gel analysis, precipitation reactions, immunofluorescence assays, protein A assays, and immunoelectrophoresis assays.

Preferred genetically-modified animal cells of the invention are embryonic stem (ES) cells and ES-like cells. These cells are derived from the preimplantation embryos and blastocysts of various species, such as mice (Evans et al., Nature 129:154-156, 1981; Martin, Proc. Natl. Acad. Sci., USA, 78: 7634-7638, 1981), pigs and sheep (Notanianni et al., J. Reprod. Fert. Suppl., 43: 255-260, 1991; Campbell et al., Nature 380: 64-68,1996) and primates, including humans (Thomson et al., U.S. Patent No. 5,843,780, Thomson et al., Science 282: 1145-1147, 1995; and Thomson et al., Proc. Natl. Acad. Sci. USA 92: 7844-7848, 1995).

These types of cells are pluripotent, that is, under proper conditions, they differentiate into a wide variety of cell types derived from all three embryonic germ layers: ectoderm, mesoderm and endoderm. Depending upon the culture conditions, a sample of ES cells can be cultured indefinitely as stem cells, allowed to differentiate into a wide variety of different cell types within a single sample, or directed to differentiate into a specific cell type, such as macrophage-like cells, neuronal cells, cardiomyocytes, chondrocytes, adipocytes, smooth muscle cells, endothelial cells, skeletal muscle cells, keratinocytes, and hematopoietic cells, such as eosinophils, mast cells, erythroid progenitor cells, or megakaryocytes. Directed differentiation is accomplished by including specific growth factors or matrix components in the culture conditions, as further described, for example, in Keller et al., Curr. Opin. Cell Biol. 7: 862-69, 1995, Li et al., Curr. Biol. 8: 971, 1998, Klug et al., J. Clin. Invest. 98: 216-24, 1996, Lieschke et al., Exp. Hematol. 23: 328-34, 1995, Yamane et al., Blood 90: 3516-23, 1997, and Hirashima et al., Blood 93: 1253-63, 1999.

The particular embryonic stem cell line that is used for genetic modification is not critical; exemplary murine ES cell lines include AB-1 (McMahon and Bradley, Cell 62:1073-85, 1990), E14 (Hooper et al., Nature 326: 292-95, 1987), D3 (Doetschman et al., J. Embryol. Exp. Morph. 87: 27-45, 1985), CCE (Robertson et al, Nature 323: 445-48, 1986), RW4 (Genome Systems, St. Louis, MO), and DBA/1lacJ (Roach, et al., Exp. Cell Res. 221: 520-25, 1995). Genetically-modified murine ES cells may be used to generate genetically-modified mice, according to published procedures (Robertson, 1987, *Teratocarcinomas and Embryonic Stem Cells: A Practical Approach*, Ed. E. J. Robertson, Oxford: IRL Press, pp. 71-112, 1987; Zjilstra et al., Nature 342: 435-438, 1989; and Schwartzberg et al., Science 246: 799-803, 1989).

Following confirmation that the ES cells contain the desired functional disruption of the GST-Omega-1 gene, these ES cells are then injected into suitable blastocyst hosts for generation of chimeric mice according to methods known in the art (Capecchi, Trends Genet. 5: 70, 1989). The particular mouse blastocysts employed in the present invention are not critical. Examples of such blastocysts include those derived from C57BL6 mice, C57BL6 Albino mice, Swiss outbred mice, CFLP mice, and MFI mice. Alternatively ES cells may be sandwiched between tetraploid embryos in aggregation wells (Nagy et al., Proc. Natl. Acad. Sci. USA90: 8424-8428, 1993).

The blastocysts or embryos containing the genetically-modified ES cells are then implanted in pseudopregnant female mice and allowed to develop *in utero* (Hogan et al., *Manipulating the Mouse Embryo: A Laboratory Manual*, Cold Spring Harbor Laboratory press, Cold Spring Harbor, NY 1988; and *Teratocarcinomas and Embryonic Stem Cells: A Practical Approach*, E.J. Robertson, ed., IRL Press, Washington, D.C., 1987). The offspring born to the foster mothers may be screened to identify those that are chimeric for the GST-Omega-1 gene disruption. Generally, such offspring contain some cells that are derived from the genetically-modified donor ES cell as well as other cells derived from the original blastocyst. In such circumstances, offspring may be screened initially for mosaic coat color, where a coat color selection strategy has been employed, to distinguish cells derived from the donor ES cell from the other cells of the blastocyst. Alternatively, DNA from tail tissue of the offspring can be used to identify mice containing the genetically-modified cells.

The mating of chimeric mice that contain the GST-Omega-1 gene disruption in germ line cells produces progeny that possess the GST-Omega-1 gene disruption in all germ line cells and somatic cells. Mice that are heterozygous for the GST-Omega-1 gene disruption can then be crossed to produce homozygotes (see, e.g., U.S. Pat. No. 5,557,032, and U.S. Pat. No. 5,532,158).

An alternative to the above-described ES cell technology for transferring a genetic modification from a cell to a whole animal is to use nuclear transfer. This method can be employed to make other genetically-modified, non-human mammals besides mice, for example, sheep (McCreath et al., Nature 29: 1066-69, 2000; Campbell et al., Nature 389: 64-66, 1996; and Schnieke et al., Science 278: 2130-33, 1997) and calves (Cibelli et al., Science 280: 1256-58, 1998). Briefly, somatic cells (e.g., fibroblasts) or pluripotent stem cells (e.g., ES-like cells) are selected as nuclear donors and are genetically-modified to contain a functional disruption of the GST-Omega-1 gene. When inserting a DNA vector into a somatic cell to mutate the GST-Omega-1 gene, it is preferred that a promoterless marker be used in the vector such that vector integration into the GST-Omega-1 gene results in expression of the marker under the control of the GST-Omega-1 gene promoter (Sedivy and Dutriaux, T.I.G. 15: 88-90, 1999; McCreath et al., Nature 29: 1066-69, 2000). Nuclei from donor cells which have the appropriate GST-Omega-1 gene disruption are then transferred to fertilized or parthenogenetic oocytes that are enucleated (Campbell et al., Nature 380: 64, 1996; Wilmut et al., Nature 385: 810, 1997). Embryos are reconstructed, cultured to develop into the morula/blastocyst stage, and transferred into foster mothers for full term *in utero* development.

The present invention also encompasses the progeny of the genetically-modified, non-human mammals and genetically-modified animal cells. While the progeny are heterozygous or homozygous for the genetic modification that disrupts the GST-Omega-1 gene, they may not be genetically identical to the parent non-human mammals and animal cells due to mutations or environmental influences, besides that of the original genetic disruption of the GST-Omega-1 gene, that may occur in succeeding generations.

The cells from a non-human genetically modified animal can be isolated from tissue or organs using techniques known to those of skill in the art. In one embodiment, the genetically modified cells of the invention are immortalized. In accordance with this embodiment, cells can be immortalized by genetically engineering the telomerase gene, an oncogene, e.g., mos or *v-src*, or an apoptosis-inhibiting gene, e.g., *bcl-2,* into the cells. Alternatively, ceiis can be immortalized by fusion with a hybridization partner utilizing techniques known to one of skill in the art.

### 6. "Humanized" Non-human Mammals and Animal Cells

The genetically-modified non-human mammals and animal cells (non-human) of the invention containing a disrupted endogenous GST-Omega-1 gene can be further modified to express the human GST-Omega-1 sequence (referred to herein as "humanized"). A preferred method for humanizing cells involves replacing the endogenous GST-Omega-1 sequence with nucleic acid sequence encoding the human GST-Omega-1 sequence (Jakobsson et al., Proc. Natl. Acad. Sci. USA 96: 7220-25, 1999) by homologous recombination. The vectors are similar to those traditionally used as targeting vectors with respect to the 5' and 3' homology arms and positive/negative selection schemes. However, the vectors also include sequence that, after recombination, either substitutes the human GST-Omega-1 coding sequence for the endogenous sequence, or effects base pair changes, exon substitutions, or codon substitutions that modify the endogenous sequence to encode the human GST-Omega-1. Once homologous recombinants have been identified, it is possible to excise any selection-based sequences (e.g., neo) by using Cre or Flp-mediated site directed recombination (Dymecki, Proc. Natl. Acad. Sci. 93: 6191-96, 1996).

When substituting the human GST-Omega-1 sequence for the endogenous sequence, it is preferred that these changes are introduced directly downstream of the endogenous translation start site. This positioning preserves the endogenous temporal and spatial expression patterns of the GST-Omega-1 gene. The human sequence can be the full length human cDNA sequence with a polyA tail attached at the 3' end for proper processing or the whole genomic sequence (Shiao et al., Transgenic Res. 8: 295-302, 1999). Further guidance regarding these methods of genetically modifying cells and non-human mammals to replace expression of an endogenous gene with its human counterpart is found, for example, in Sullivan et al., J. Biol. Chem. 272: 17972-80, 1997, Reaume et al., J. Biol. Chem. 271: 23380-88, 1996, and Scott et al., U.S. Pat. No. 5,777,194).

Another method for creating such "humanized" organisms is a two step process involving the disruption of the endogenous gene followed by the introduction of a transgene encoding the human sequence by pronuclear microinjection into the knock-out embryos.

### 7. Uses for the Genetically-Modified Non-human Mammals and Animal Cells

GST-Omega-1 function and therapeutic relevance can be elucidated by investigating the phenotype of the non-human mammals and animals cells of the invention that are homozygous (-/-) and heterozygous (+/-) for the disruption of the GST-Omega-1 gene. For example, the genetically-modified GST-Omega-1 -/- non-human mammals and animal cells can be used to determine whether the GST-Omega-1 plays a role in causing or preventing symptoms or phenotypes to develop in certain models of disease, e.g., arthritis or cancer. If a symptom or phenotype is different in a GST-Omega-1 -/- non-human mammal or animal cell as compared to a wild type (GST-Omega-1 +/+) or GST-Omega-1 +/- non-human mammal or animal cell, then the GST-Omega-1 polypeptide plays a role in regulating functions associated with the symptom or phenotype. Examples of animal models that can be used to assess GST-Omega-1 function include models to assess inflammation, e.g., collagen-induced arthritis (Bliver et al., Arthritis and Rheumatism 29: 1131-38, 1986; Griffiths et al., J. Expt. Med. 185: 1123-29, 1997; and Joosten et al., J. Immunol. 163: 5049-55, 1999), concanavalin A-induced inflammation (Hentze et al., Am. J. Pathol. 156: 2045-56, 2000; and Kim et al., Clin. Immunol. 97: 221-33, 2000), and anti-collagen monoclonal antibody-induced arthritis (Chemicon International Inc., Temecula, CA).

In addition, under circumstances in which an agent has been identified as a GST-Omega-1 agonist or antagonist (e.g., the agent significantly modifies one or more of the GST-Omega-1 polypeptide activities when the agent is administered to a GST-Omega-1 +/+ or GST-Omega-1 +/- non-human mammal or animal cell), the genetically-modified GST-Omega-1 -/- non-human mammals and animal cells of the invention are useful to characterize any other effects caused by the agent besides those known to result from the (ant)agonism of GST-Omega-1 (i.e., the non-human mammals and animal cells can be used as negative controls). For example, if the administration of the agent causes an effect in a GST-Omega-1 +/+ non-human mammal or animal cell that is not known to be associated with GST-Omega-1 polypeptide activity, then one can determine whether the agent exerts this effect solely or primarily through modulation of GST-Omega-1 by administering the agent to a corresponding GST-Omega-1 -/- non-human mammal or animal cell. If this effect is absent, or is significantly reduced, in the GST-Omega-1 -/- non-human mammal or animal cell, then the effect is mediated, at least in part, by GST-Omega-1. However, if the GST-Omega-1 -/- non-human mammal or animal cell exhibits the effect to a degree comparable to the GST-Omega-1 +/+ or GST-Omega-1 +/- non-human mammal or animal cell, then the effect is mediated by a pathway that does not involve GST-Omega-1 signaling.

Furthermore, if an agent is suspected of possibly exerting an effect via a GST-Omega-1 pathway, then the GST-Omega-1-/- non-human mammals and animal cells are useful as negative controls to test this hypothesis. If the agent is indeed acting through GST-Omega-1, then the GST-Omega-1-/- non-human mammals and animal cells, upon administration of the agent, should not demonstrate the same effect observed in the GST-Omega-1 +/+ non-human mammals or animal cells.

The genetically modified non-human mammals and animal cells of the invention can also be used to identify genes whose expression is upregulated in GST-Omega-1 +/- or GST-Omega-1 -/- non-human mammals or animal cells relative to their respective wild-type control. Techniques known to those of skill in the art can be used to identify such genes based upon the present description. For example, DNA assays can be used to identify genes whose expression is upregulated in GST-Omega-1 +/- or GST-Omega-1 -/- mice to compensate for a deficiency in GST-Omega-1 expression. DNA arrays are known to those of skill in the art (see, e.g., Aigner et al., Arthritis and Rheumatism 44: 2777-89, 2001; U.S. Patent No. 5,965,352; Schena et al., Science 270: 467-470, 1995; DeRisi et al., Nature Genetics 14: 457-460, 1996; Shalon et al., Genome Res. 6: 639-645, 1996; and Schena et al., Proc. Natl. Acad. Sci. (USA) 93: 10539-11286,1995).

### 8. Therapeutic Methods

Agents identified as GST-Omega-1 inhibitors are administered in a dose sufficient to reduce IL-1 levels (preferably by more than 50%), and/or IL-1 mediated pathology (e.g., joint inflammation, joint swelling, joint ankylosis, and/or joint immobility). Such therapeutically effective amounts will be determined using routine optimization techniques that are dependent on the particular condition to be treated, the condition of the patient, the route of administration, the formulation, the judgment of the practitioner, and other factors evident to those skilled in the art in light of this disclosure.

Agents that are GST-Omega-1 inhibitors are useful to treat and/or reduce symptoms of IL-1 mediated or inflammation-mediated disorders such as osteoarthritis, rheumatoid arthritis, graft vs. host (transplant rejection), thrombocytopenia, acquired hemolytic anemia, erythroblastopenia, inflammatory bowel diseases, multiple sclerosis, lupus, drug and food allergies, ophthamalic diseases, including allergic conjunctivitis and keratitis, asthma, atopic dermatitis, contact dermatitis, chronic obstructive pulmonary disease, acute respiratory distress syndrome, IL-1 dependent cancers (e.g., myeloma), psoriasis, dermatitis, rheumatic carditis, bursitis, psoriatic arthritis, and glucocorticoid insufficiency.

GST-Omega-1 inhibitors may be coadministered with methotrexate, analgesics (e.g. NSAIDS), disease modifying agents (e.g., CSAIDS), penicillamine, colloidal gold, phosphodiesterase inhibitors, cyclosporin, FK 506, biological inhibitors of TNFa or its receptor or IL-1 or its receptor (e.g. Enbrel or Remicade or Kineret), metalloprotease inhibitors, bronchodilators, antihistamines, pyrimidine synthesis inhibitors (leflunomide).

An agent that inhibits GST-Omega-1 activity can be incorporated into a therapeutic composition. Such GST-Omega-1 inhibitors can include small molecules, nucleic acids, e.g., GST-Omega-1 antisense nucleic acids, amino acids, peptides, and carbohydrates. Preferably, such agents are combined with a pharmaceutically acceptable delivery vehicle or carrier.

As used herein, a pharmaceutically acceptable delivery vehicle includes solvents, dispersion media, coatings, antibacterial and antifungal agents, and isotonic and absorption delaying agents that are compatible with pharmaceutical administration. The vehicle may also include other active or inert components, and/or may be targeted to joint tissue by virtue of its composition.

A therapeutic composition is formulated to be compatible with its intended route of administration. Non-limiting examples of routes of administration include parenteral, e.g., intravenous, intradermal, subcutaneous, oral (e.g., by ingestion or inhalation), transdermal (topical), transmucosal, and rectal administration. Solutions or suspensions can be made as described in *Remington's Pharmaceutical Sciences*, (18^{th} ed., Gennaro, ed., Mack Publishing Co., Easton, PA, (1990)).

Therapeutic efficacy of such GST-Omega-1 inhibitors can be determined in light of this disclosure by standard therapeutic procedures in cell cultures or experimental animals, e.g., for determining the ED₅₀ (the dose therapeutically effective in 50% of the population).

The data obtained from the cell culture assays and animal studies can be used in formulating a range of dosage for use in humans. The dosage may vary depending upon the formulation and the route of administration. For any GST-Omega-1 inhibitor used in the method of the invention, the therapeutically effective dose can be estimated initially from cell culture assays. A dose may be formulated in animal models to achieve a circulating plasma concentration range that includes the IC₅₀ as determined in cell culture. Such information can be used to more accurately determine useful doses in humans. Levels in plasma may be measured, for example, by high performance liquid chromatography.

The skilled artisan will appreciate that certain factors may influence the dosage and timing required to effectively treat a mammal including, but not limited to, the severity of the disease or disorder, previous treatments, the general health and/or age of the mammal, and other diseases present. Moreover, treatment of a mammal with a therapeutically effective amount of an GST-Omega-1 inhibitor can include a single treatment or can include a series of treatments.

GST-Omega-1 inhibitors (e.g., antagonists) that can be administered include those described in U.S. Pat. No. 6,166,064 and in Perregaux et al., J. Pharm. Exp. Therap. 299: 187-97, 2001.

### Examples

### A. Targeting Vector Construction

A 700 bp murine GST-Omega-1 genomic PCR fragment was used as a probe to identify genomic clones from a DBA/1 LacJ genomic phage library (Stratagene, La Jolla, CA).

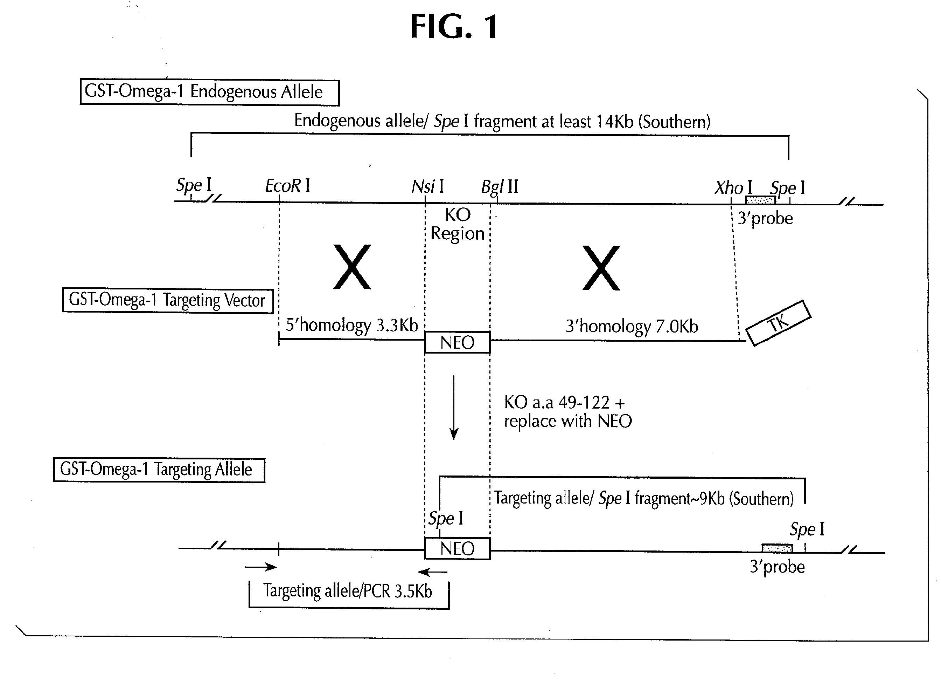

The GST-Omega-1 targeting vector was constructed by cloning a 3.3 kb fragment from a genomic clone for use as the 5' homology arm and a 7.0 kb fragment from a genomic clone for use as the 3' homology arm. These fragments were isolated and cloned into a pJNS2 (PGK-NEO/PGK-TK) targeting vector backbone (Dombrowicz et al., Cell 75: 969-76, 1993). The final targeting vector clone was designed to replace approximately 1.8 kb of GST-Omega-1 genomic sequence with the neomycin gene. This deletion of the GST-Omega-1 sequence created a deletion of amino acids 49 to 102 in the GST-Ω-1 polypeptide sequence (Fig. 1).

### B. ES Cell Screening

The GST-Omega-1 KO targeting vector was linearized and electroporated into DBA/252 ES cells (Roach et al., Exp. Cell. Res. 221: 520-25, 1995). The DBA/252 ES cells were maintained in stem cell medium (SCML) which consisted of knockout DMEM (Invitrogen Life Technologies, Inc. (ILTI), Carlsbad, CA, #10828-018) supplemented with 15% heat inactivated ES cell qualified fetal bovine serum (ILTI, #10439-024), 0.1 mM 2-mercaptoethanol (Sigma Chemical, St. Louis, MO, #M-7522), 0.2 mM L-glutamine (ILTI, #25030-081), 0.1 mM MEM non-essential amino acids (ILTI, #11140-050), 400 units/ml recombinant murine leukemia inhibitory factor (Chemicon International Inc., Temecula, CA, # ESG-1107) and penicillin/streptomycin (ILTI, #15140-122).

Electroporation of 1X10⁸ cells in SCML was carried out using a BTX Electro Cell Manipulator 600 (BTX, Inc., San Diego, CA) at a voltage of 200 V, a capacitance of 50 µF, and a resistance of 360 Ohms. Positive/negative selection was then conducted in SCML which contained 200 µg/ml G418 (ILTI, #11811-031) and 2 µM gancyclovir (Syntex Laboratories, Palo Alto, CA) as previously described (Mansour et al, Nature 236: 348-52, 1988).

DNA was isolated from ES cell clones which survived G418 and gancyclovir selection. The digests were electrophoresed on 0.7% agarose gels (BioWhittaker Molecular Applications, Rockland, ME) and transferred to Hybond N+ (Amersham Pharmacia Biotech, Buckinghamshire, England) nylon membrane for Southern analysis.

A 700 bp probe designed to hybridize to a region downstream of the 7.0 kb 3' homology arm was used as a probe to screen for homologous recombination on the 3' side in Spel-digested ES cell DNA. The wild type allele yielded a band hybridizing at > 12 kb and the targeted allele yielded a band hybridizing at about 9.5 kb. Gene targeting at the 5' end was confirmed by long PCR with a forward primer from a region upstream of the 5' homology arm (5'-AGGCTATGGCTTGGTCAGGC-3') (SEQ ID NO: 1) and a reverse primer in the neomycin promoter of the targeting vector (5'-TGCTACTTCCATTTGTCACGTCC-3') (SEQ ID NO: 2). Homologously targeted ES cell clones were also screened for the incidence of multiple insertions by Southern analysis using a neo probe.

### C. Knockout Mouse Production

ES cells with a targeted disruption of the GST-Omega-1 gene were microinjected into blastocyst stage embryos isolated from CS7BL/6J females (The Jackson Laboratory, Bar Harbor, ME).

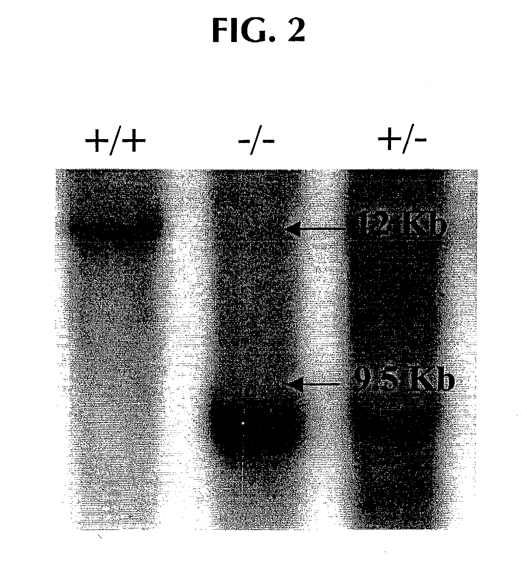

Male chimeras for both clones were identified and back-crossed to DBA/1lacJ females (The Jackson Laboratory) to generate germline GST-Omega-1 heterozygous (+/-) offspring. Homozygous GST-Omega-1 knockout mice (GST-Omega-1 KO) were generated from heterozygous matings (heterozygous x heterozygous) with normal Mendelian ratios observed. Genotypes were confirmed by Southern analysis of DNA samples (Fig. 2).

Histological examination of tissues and organs from wild type and GST-Omega-1 knockout mice demonstrated no overt changes resulting from homozygous disruption of the GST-Omega-1 gene.

### D. GST-Omega-1 Expression

RT-PCR was performed on 1 µg/ml of liver, heart, spleen, and kidney samples of total RNA in a 20 µl reaction mixture containing the following: 500 µM each of dATP, dUTP, dCTP, and dGTP (ILTI); 1X PCR buffer (ILTI); 2.5 mM MgCl₂ (ILTI) 0.01 M dithiothreitol (DTT) (ILTI); and 200 U of Superscipt™ II reverse transcriptase (ILTI). The reverse transcription reaction was carried out for 1 hour at 42°C; the denaturation reaction was carried out at 70°C for 15 min. Aliquots (2 µl) of each reaction mixture were then amplified in a 50 µl reaction mixture containing: 1X PCR buffer, 1.4 mM MgCl₂, 200 µM each of dATP, dUTP, dCTP, and dGTP; 0.4 µM of each of the primers further discussed below; and 1U Taq polymerase (Roche-Boehringer Mannheim, Indianapolis, IN). PCR cycling conditions were 30 cycles at: 94°C for 30 sec., 55°C for 30 sec., and 72°C for 30 sec. Amplification of the β-actin gene was carried out as a positive control. RT PCR was also performed on all RNA samples in the absence of reverse transcriptase as an additional control.

For RT-PCR analysis of GST-Omega-1, the primers used were

5'-ACGAAGTCATCAACATCAACC-3' (SEQ ID NO: 3); and

5'-TAACTTCTTCTCTGGGTAGGC-3' (SEQ ID NO: 4).

For PCR analysis of β-actin, the primers used were:

5'-GTGGGCCGCTCTAGGCACCAA-3' (SEQ ID NO: 5); and

5'-CTCTTTGATGTCACGCACGATTTC-3' (SEQ ID NO: 6).

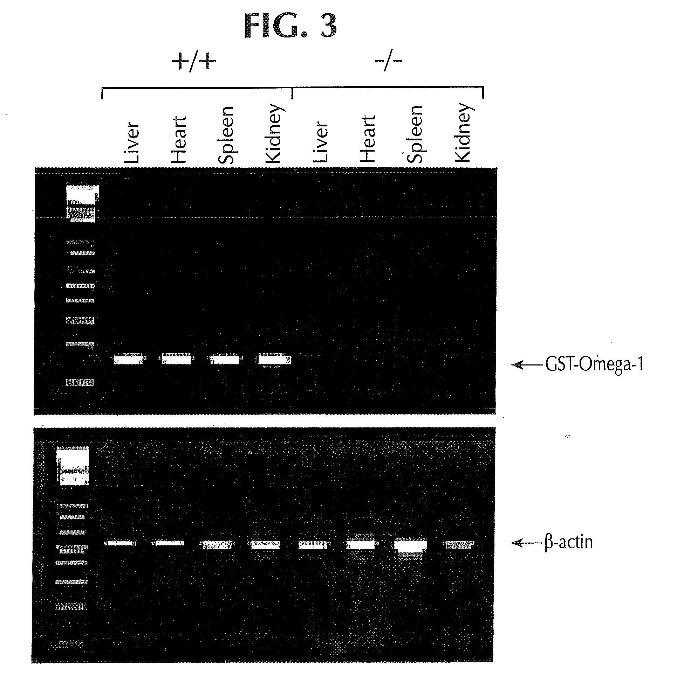

All of these primer sequences were prescreened against the BLASTN sequence similarity database to ensure that no other murine genes would cross hybridize to these primers (newblast@ncbi.nlm.nih.gov). PCR products were separated by electrophoresis on a 2% agarose gel and visualized by ethidium bromide staining (Figure 3). The results confirmed that GST-Omega-1 was expressed in all wild type tissues studied and was not expressed in any knockout tissues studied.

### E. Effects on Collagen-Induced Arthritis

On day 0, wild type and GST-Omega-1 knockout mice were injected intradermally at the base of the tail with 100 µl of an emulsion containing 50 µl of Freund's complete adjuvant (made from Freund's incomplete adjuvant (Sigma Chemical) with 2 mg/ml *Mycobacterium tuberculosis* (Difco Labs, Detroit, Ml)) and 50 µl of 2 mg/ml type II chick collagen (Chondrex, Redmond, WA) in 0.1 M acetic acid (Sigma Chemical). On day 21, this immunization was repeated, and the mice were assessed for the development of arthritis by the degree of swelling, redness, and ankylosis of the joints until day 40. All visual factors were combined into a score of 0-3 per paw and summed for a total score of 0-12 for each animal.

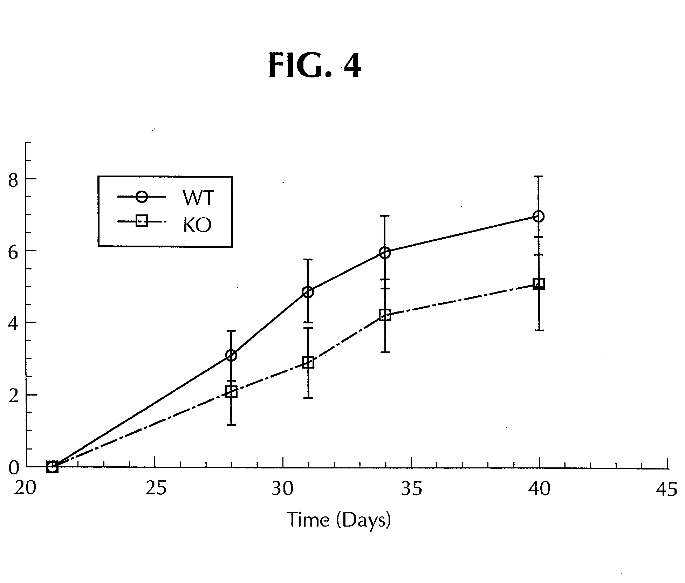

As shown in Fig. 4, the GST-Omega-1 knockout mice demonstrated reduced levels of collagen induced arthritis at each time point following immunization.

The wild type and GST-Omega-1 knockout animals were then euthanized by CO₂ asphyxiation, and stifle (knee) sections were subjected to a comprehensive histological assessment that included evaluations of cartilage structure, cellularity, Safranin-O staining for acid mucopolysaccharides, and synovial inflammation and hyperplasia to develop Modified Mankin scores (Mankin, et al., J. Bone and Joint Surgery 53: 523-537, 1971). Originally developed to quantify changes in articular cartilage in humans with osteoarthritis, the Mankin scale used herein was modified for rodents to reflect rodent size and to include synovial inflammation.

The Mankin scores were based upon a scale of 0-4 per joint graded on a relative severity scale (with 4 designated for the most severe form of arthritic symptoms), for a maximum score of 16 per animal. As shown in Table 1, the Mankin scores also demonstrated a reduced level of joint inflammation in the GST-Omega-1 KO mice as compared to wild type mice.

**Table 1.**

| **Modified Mankin Scores (mean ± S.D.)** | |
|---|---|
| Untreated | 1.8 ± 0.3 |
| GST-Omega-1 KO + collagen | 4.4 ± 3.1 |
| Wild type + collagen | 10.8 ± 3.9 |

Therefore, the reduced level of arthritic symptoms displayed by GST-Omega-1 KO mice of the invention, when exposed to an experimental model of arthritis, demonstrate that administering an agent that inhibits GST-Omega-1 activity can be an effective means of reducing IL-1 mediated symptoms, and can be used to treat IL-1 and inflammation-mediated disorders.

### G. Effects on Concanavalin A-Induced Liver Injury

Concanavalin A (type V from *Canavalia ensiforms*) resuspended in 0.9% NaCI injection saline (B. Braun Medical Inc., Irvine, CA) in doses of 0.3 mg/kg and 3.0 mg/kg, was injected intravenously in GST-Omega-1 KO and wild type mice. After 8 hours, the mice were sacrificed and serum was analyzed to assess liver enzyme level using an automated analyzer. With the 3.0 mg/kg dose, alanine transaminase (ALT), aspartate transaminase (AST), and sorbitol dehydrogenase (SDH) were 2.7, 3.6, and 1.8 fold lower in the KO mice relative to the wild type controls, demonstrating that the KO mice exhibited less inflammation-induced liver injury.

As with the collagen-induced arthritis model discussed above, the concanavalin-induced liver injury model showed that the GST-Omega-1 KO mice of the invention had reduced symptoms of inflammation. These liver injury results confirm that administering a GST-Omega-1 inhibitor can be an effective means to reduce inflammation and to treat IL-1 and inflammation-related disorders.

## Claims

1. A genetically-modified, non-human mammal, wherein the modification results in a disrupted glutathione S-transferase-Omega-1 (GST-Omega-1) gene.

2. The mammal of claim 1, wherein said mammal is a rodent.

3. The rodent of claim 2, wherein said rodent is a mouse.

4. The non-human mammal of claim 1, wherein said mammal is homozygous for said modification.

5. A genetically-modified animal cell, wherein the modification comprises a disrupted GST-Omega-1 gene.

6. The animal cell of claim 5, wherein said cell is an embryonic stem (ES) cell or an ES-like cell.

7. The animal cell of claim 5, wherein said cell is isolated from a genetically-modified, non-human mammal containing a modification that results in a disrupted GST-Omega-1 gene.

8. The animal cell of any one of claims 5, 6 or 7, wherein said cell is murine.

9. The animal cell of claim 5, wherein said cell is human.

10. The animal cell of any one of claims 5 to 9, wherein said cell is homozygous for said modification.

11. A method of identifying a gene that demonstrates modified expression as a result of modified GST-Omega-1 activity in an animal cell, said method comprising comparing the expression profile of a genetically modified animal cell, wherein the cell is homozygous for a genetic modification that disrupts the GST-Omega-1 gene, to a wild type cell.

12. Use of an agent that inhibits GST-Omega-1 activity in the manufacture of a medicament for the treatment of an IL-1 mediated and/or inflammation mediated disorder in a mammal.

13. The use of claim 12, wherein the disorder is rheumatoid arthritis, osteoarthritis, inflammatory bowel disorder, osteoporosis, or IL-1 dependent cancer.


SEQUENCE LISTING

<110>  Pfizer Products Inc.

<120>  DISRUPTION OF THE GLUTATHIONE S-TRANSFERASE-OMEGA-1 GENE

<130>  PC23128A

<150>  US 60/341,483

<151>  2001-12-14

<160>  6

<170>  PatentIn version 3.1

<210>  1

<211>  20

<212>  DNA

<213>  Mus musculus

<400>  1
aggctatggc ttggtcaggc                                                    20

<210>  2

<211>  23

<212>  DNA

<213>  Mus musculus

<400>  2
tgctacttcc atttgtcacg tcc                                                23

<210>  3

<211>  21

<212>  DNA

<213>  Mus musculus

<400>  3
acgaagtcat caacatcaac c                                                21

<210>  4

<211>  21

<212>  DNA

<213>  Mus musculus

<400>  4
taacttcttc tctgggtagg c                                                21

<210>  5

<211>  21

<212>  DNA

<213>  Mus musculus

<400>  5
gtgggccgct ctaggcacca a                                                21

<210>  6

<211>  24

<212>  DNA

<213>  Mus musculus

<400>  6
ctctttgatg tcacgcacga tttc                                             24